# EUROPEAN PATENT SPECIFICATION

(11) **EP 4 384 249 B1**
(45) Date of publication and mention of the grant of the patent: **18.06.2025**
(21) Application number: 22761907.9
(22) Date of filing: 04.08.2022
(51) Int. Cl.: A61M 25/00, B65D 33/25

(54) **HYGIENIC DISPOSAL CATHETER PRODUCT**
KATHETERPRODUKT ZUR HYGIENISCHEN ENTSORGUNG
PRODUIT DE CATHÉTER D'ÉLIMINATION HYGIÉNIQUE

(30) Priority: 11.08.2021 US 202163231857 P
(43) Date of publication of application: 19.06.2024
(60) Divisional application: 25174999.0
(73) Proprietor: Hollister Incorporated, Libertyville, IL 60048-3781 (US)
(72) Inventor: DOHERTY, John, Patrick, Libertyville, IL 60048 (US)
(74) Representative: FRKelly
(86) International application number: PCT/US2022/074548
(87) International publication number: WO 2023/019079

(56) References cited:
- EP-A1- 2 873 627
- WO-A1-2014/142895
- WO-A1-2018/190865
- WO-A1-2019/148454
- GB-A- 2 579 273
- US-A1- 2007 110 340

## Description

### BACKGROUND

### FIELD OF THE DISCLOSURE

The present disclosure generally relates to catheter products that include a package for easy and convenient disposal of the product after use. More particularly, the present disclosure relates to intermittent urinary catheter products including a package having an associated reclosable member.

### DESCRIPTION OF RELATED ART

Certain medical devices, such as intermittent urinary catheters, are commonly used by those who suffer from various abnormalities of the urinary system, such as urinary incontinence. Urinary catheters generally comprise a shaft portion with two ends. A first end has a catheter tip that is inserted into a user's urethra. A second end generally has a funnel that is used to help facilitate and direct urine drainage.

These urinary catheters are usually stored in a package that is openable but not reclosable. Typically, a user opens the package, throws the package away, uses the catheter, and then disposes the catheter separately from the package. During disposal of the catheter after use urine may leak from the spent catheter, creating an undesirable mess. Accordingly, there is a need for an improved intermittent urinary catheter product as shown and described herein.

WO2014142895 discloses one of such catheters.

### SUMMARY

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, an intermittent urinary catheter product is disclosed. The catheter product includes a package having opposed front and rear panels that are sealed together to define a sealed interior cavity. The package has top and bottom edges and opposed side edges. The front panel has an inner surface, and the rear panel has an inner surface. At least one of the inner surface of the front panel and the inner surface of the rear panel includes a reclosable member associated therewith. The reclosable member has a longitudinal axis that is generally parallel with one of the edges. The front and rear panels of the package are tearable adjacent to the reclosable member and in a direction parallel to the reclosable member. The tearing of the front and rear panels forms an opening in the package adjacent to the reclosable member and in communication with the interior. The reclosable member is configured to attach the inner surface of the front panel to the inner surface of the rear panel so as to close the opening. A hydration fluid is contained within the cavity and a hydrophilic urinary catheter is contained within the cavity.

In another aspect, a package for use in an intermittent urinary catheter product is disclosed. The package includes a front panel and a rear panel. The front panel is opposed to the rear panel and the front and rear panels are sealed together to define a sealed interior cavity. The package also has a top edge, a bottom edge, a first side edge and a second side edge and the second side edge is opposed to the first side edge. The front panel has an inner surface, and the rear panel has an inner surface. At least one of the inner surface of the front panel and the inner surface of the rear panel includes a reclosable member associated therewith. The reclosable member has a longitudinal axis that is generally parallel with one of the edges. The front and rear panels of the package are tearable adjacent to the reclosable member and in a direction parallel to the reclosable member. Tearing of the front and rear panels forms an opening in the package adjacent to the reclosable member and in communication with the interior. The reclosable member is configured to attach to the inner surface of the front panel to the inner surface of the rear panel so as to close the opening. The cavity is configured to contain a hydration fluid and a hydrophilic urinary catheter.

### BRIEF DESCRIPTION OF THE DRAWINGS

Fig. 1 is a front perspective view of an intermittent urinary catheter product;
Fig. 2 is a rear perspective view of the intermittent urinary catheter product of Fig. 1;
Fig. 3 is a perspective view of the intermittent urinary catheter product of Fig. 1, showing the product in an open configuration, such that an opening is visible;
Fig. 4 is a side plan view of the intermittent urinary catheter product of Fig. 3, showing the opening of the package;
Fig. 5 is a partial cross-sectional view of the intermittent urinary catheter product of Fig. 1, taken along line V-V and showing the reclosable member associated with the opening of the present invention;
Fig. 6 is a partial cross-sectional view of the intermittent urinary catheter product of Fig. 1, taken along line V-V and showing a first example of a reclosable member associated with the opening, the first example including a sealing strip;
Fig. 7 is a partial cross-sectional view of the opening of the intermittent urinary catheter product of Fig. 6, showing the sealing strip being peeled;
Fig. 8 is a cross-sectional view of the intermittent urinary catheter product of Fig. 1, showing a hydrophilic urinary catheter and a hydration fluid contained within the package; and
Fig. 9 is a perspective view of the intermittent urinary catheter product of Fig. 8, showing the package reclosed after the catheter has been placed back into the package after use.

### DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims. The invention is defined in claim 1.

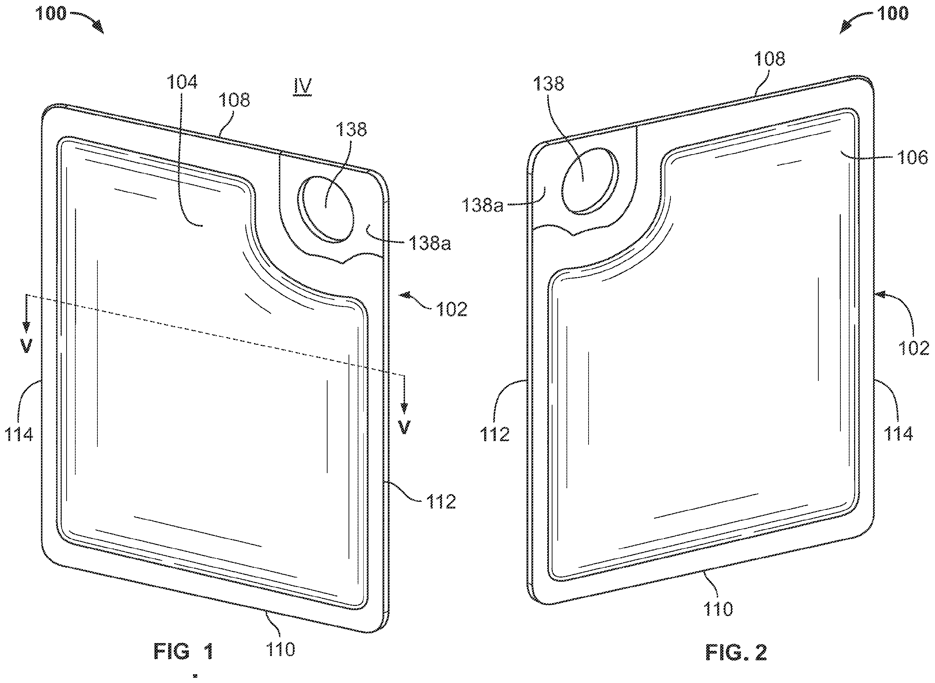

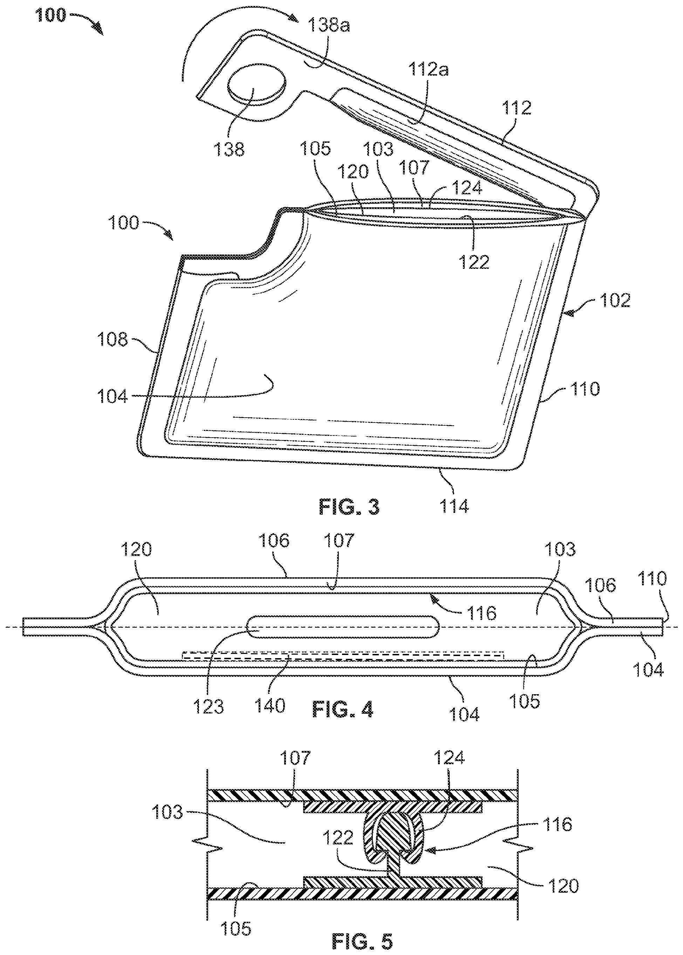

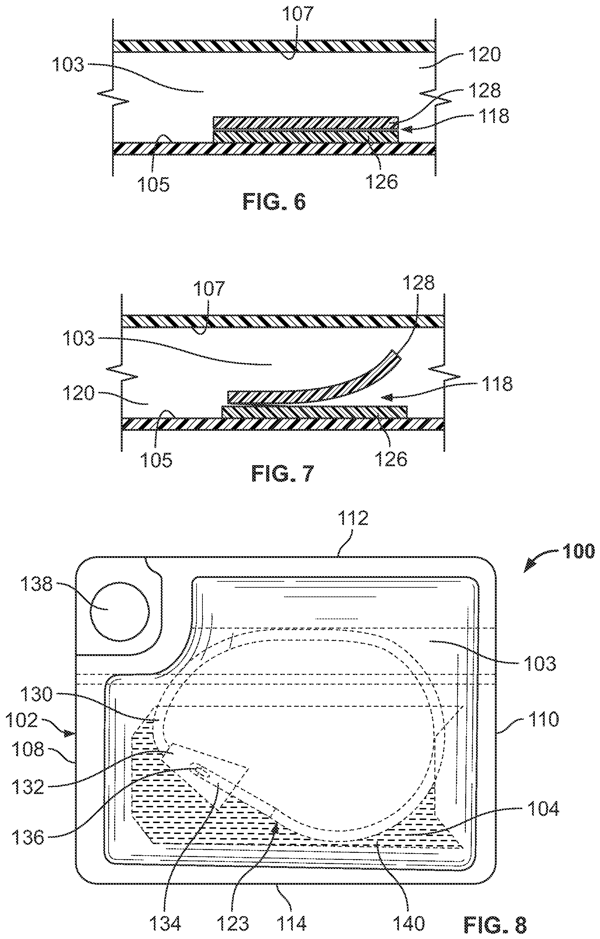

Figs. 1-2 illustrate an embodiment of an intermittent urinary catheter product 100 including a package 102, which may be generally rectangular or any other suitable shape. For example, the package may be sustainably round or oval, or may be a polygonal shape. The package 102 has opposed front and rear panels (104 and 106, respectively) that are sealed together to define a sealed interior cavity 103 (Figs. 3-5). The panels 104, 106 may be sealed together by any suitable method such as heat sealing. The sealed interior cavity 103 may be airtight and watertight when closed such that it is suitable for sterilely containing an intermittent urinary catheter 123 (Figs. 4 and 8). The package 102 has top and bottom edges (108 and 110, respectively) and opposed side edges (112 and 114, respectively).

As shown in Figs. 3-4, the package 102 is tearable such that an opening 120 is formed in the package 102 when a user tears the package 102. The opening 120 is in communication with the cavity 103 and allows access to the urinary catheter 123 within the cavity 103. The package 102 may include tear direction element(s) associated with the package 102 to facilitate tearing in a desired direction, such as in a substantially straight line adjacent and parallel to an edge of the package 102. The tear elements may be tear tape, tear strip(s) or may be the material of the panels. For example, the material of the panels may include an orientated polymer that directs tearing of the panels in a substantially straight line in a desired direction. As shown, the front panel 104 has an inner surface 105, and the rear panel 106 has an inner surface 107. The inner surfaces 105, 107 define the cavity 103. Each panel 104, 106 may include a tear element that promotes tearing of each panel parallel and adjacent to edge 112. In the illustrated embodiment, the package 102 is tearable parallel to the side edge 112. However, in alternative embodiments the package 102 and the panels 104, 106 may be tearable parallel to the other edges.

As shown in Figs 1-3, the package 102 may include a gripping member 138, such as a finger hole to help the user tear the package 102. The gripping member 138 may be in a sealed portion 138a of the package 102. To tear the package 102, the user may place a finger in the gripping member 138 and pull such that the front and rear panels 104, 106 of the package tear parallel to edge 112, exposing the opening 120 and allowing access to the cavity 103. The torn portion 112a (Fig. 3) of the package 102 may be torn completely off such that the portion 112a may be removed after tearing. In alternative embodiments, the torn portion 112a may remain attached to the package 102. The package 102 and its parts may be made of any suitable flexible, and sealable material including but not limited to plastic or foil.

Fig. 5 shows the opening of the package 102. As shown in this figure, at least one of the inner surface 105 of the front panel 104 and the inner surface 107 of the rear panel 106 includes a reclosable member 116 associated therewith. The reclosable member 116 has a longitudinal axis A (Fig. 4) that is generally parallel with at least one of the edges, and in the illustrated embodiment side edge 112. The front and rear panels 104, 106 of the package 102 are tearable adjacent to the reclosable member 116. Thus, the front and rear panels 104, 106 are tearable between the edge 112 of the package 102 and the reclosable member 116. Accordingly, the tearing of the front and rear panels 104, 106 forms the opening 120 in the package 102 adjacent to the reclosable member 116 and in communication with the interior cavity 103.

The reclosable member 116 is configured to provide a mechanism for closing the package 102 after the user has opened the package 102. The reclosable member 116 is configured to attach the inner surface 105 of the front panel 104 to the inner surface 107 of the rear panel 106 so as to close the opening 120 with or without a watertight seal. Optionally, the reclosable member 116 provides a watertight seal for closing the opening 120.

In the invention as shown in Fig. 5, the reclosable member 116 includes a male member 122 and a female member 124. In the invention, the male member 122 is associated with inner surface 105 of front panel 104 and the female member 124 is associated with the inner surface 107 of the rear panel. The male and female members 122, 124 may be attached the inner surface in any suitable manner, such as by heat sealing or adhesive. The male member 122 and the female member 124 are configured to detachably interlock. For example, the male member 122 may be configured to interlock with the female member 124 in a push fit or an interference fit. In the invention, the male member 122 is a ridge, and the female member 124 is a groove, where the groove is configured to receive the ridge. Though a ridge and groove are shown, not in the scope of the invention, any other appropriate known mechanism for opening and closing the package 102 may be used to form the reclosable member. In order to close the reclosable member 116, the user may press the male member 122 into the female member 124 and to open the reclosable member 116, the user may disconnect the male member 122 from the female member 124 by pulling the male member 122 in a first direction and pulling the female member 124 in an opposite direction such that the male member 122 and female member 124 are disengaged. As shown, the male member 122 may be on an opposing inner surface from the female member 124, so that the user may pull one inner surface 105 away from the other inner surface 107 to disengage the male member 122 from the female member 124. The male member 122 and female member 124 may span any appropriate length of the front panel inner surface 105 and the rear panel inner surface 107, respectively. In one embodiment, the male member 122 and female member 124 span a length so as to substantially close the opening 120, and in one alternative to form a liquid-tight seal.

In an embodiment the male member and female member may span the entire length of the package 102 in a continuous manner, such as a zip-lock type configuration, where the user presses the male member into the female member in a continuous fashion. In other embodiments the male and female member may be configured in a discontinuous formation such that a user may press the male member into the female member at various points along the inner surfaces 105, 107. The male and female members may be made of any suitable material including polyethylene terephthalate and other plastic polymers.

Not forming part of the invention, figs. 6 and 7 show an alternative reclosable member 118, where the reclosable member 118 includes a sealing strip 126. The sealing strip 126 may be a double-sided adhesive wherein one side of the adhesive is initially adhered to the inner surface of a panel of the package 102. As shown, the sealing strip 126 may be positioned on the front panel inner surface 105. The sealing strip 126 may include a removable covering 128, such as a release liner, that covers the other side of the adhesive prior to use. As shown in Fig. 7, a user may pull the removable covering 128 away from the sealing strip 126 to expose the adhesive. The removable covering 128 prevents the adhesive from prematurely causing the two panels 104, 106 to stick together. When the user is ready to reclose the opening 120, the user may remove the covering 128 from the sealing strip 126, exposing the adhesive, and press the sealing strip 126 against the rear panel inner surface 107. When the user presses the adhesive covered surface of the sealing strip 126 to the rear panel inner surface 107, the sealing strip 126 sticks to the rear panel inner surface 107, closing the opening 120 and resealing the cavity 103. The adhesive may be made out of any suitable material for reclosing the package 102, such as a glue or an adhesive tape. Preferably the adhesive will be liquid resistant, such that if liquid touches the adhesive, the adhesive will still retain its adhesive properties. Though in the example shown the reclosable member 118 is on the front panel inner surface 105, in alternative examples, the reclosable member may be configured such that a part of the reclosable member is on the front panel inner surface 105 and another part of the reclosable member 118 is on the rear panel inner surface 107. In an example, the sealing strip 126 and removable covering 128 may run continuously along the front and/or rear inner surfaces of the package 102. Alternatively, the sealing strip 126 and removable covering 128 may be configured in a discontinuous fashion.

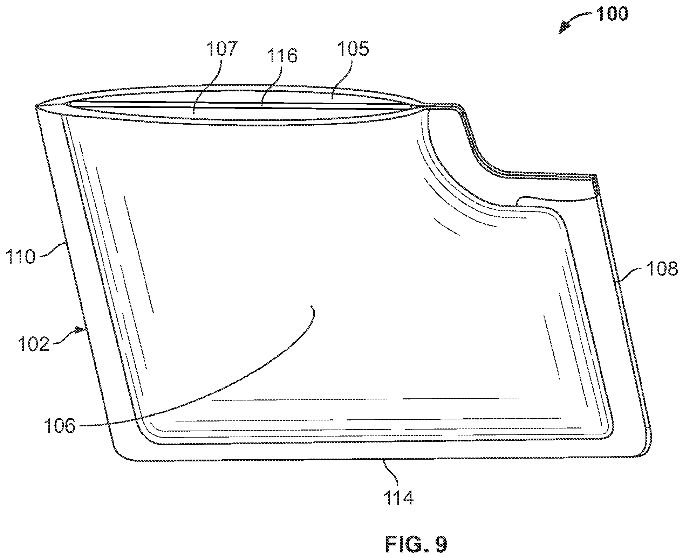

As shown, in Figs. 8 and 9, a hydrophilic urinary catheter 123 is contained within the cavity 103 of the package 102. A hydration fluid 140 is also contained within the cavity 103 such that the hydration fluid 140 hydrates the urinary catheter 123 when the urinary catheter 123 is stored in the package 102. The hydrated catheter 123 is thus ready for use when the user opens the package 102. The hydration fluid may be any suitable fluid for hydrating the catheter 123 including water. The catheter 123 has a distal end 130 including a funnel 132 and a proximal end 134 including a tip 136. The tip 136, optionally, may be configured to detachably interconnect with an interior portion of the funnel 132. The tip 136 and funnel 132 may be connected, for example, in an interference fit or push fit. As shown, the catheter 123 may form a closed loop when the tip 136 and funnel 132 are interconnected. In other embodiments, the catheter 123 may be in a straight configuration. For example, shorter female catheters may be in a straight configuration. When the catheter 123 is stored in the cavity 103, the tip 136 and funnel 132 may, but do not necessarily, interconnect in the closed loop configuration for more compact storage. When the user is ready to use the catheter 123, the user tears the package 102 open. After the package 102 is torn open, the user then may remove the catheter 123 from the package 102, detach the tip 136 from the funnel 132, if so attached, and use the catheter 123. Referring to Fig. 9, after use, when ready to dispose of the catheter 123, the user may place the catheter 123 back into the package 102 through opening 120 (Fig 3 - 7). If the catheter is so designed, the user may reattach the tip 136 to the funnel 132, such that the catheter is again in the closed-loop configuration and replace the closed-loop catheter 123 into the cavity 103 of the package 102. The catheter 123 may be placed in the package 102 such that the entirety of the catheter 123 fits into the cavity 103 below the reclosable member. The user may then close the reclosable member, resealing the package 102, as described above. After reclosing, the user may dispose of the product 100 by placing it in a trash receptacle.

Other variations and combinations may also be employed without departing from the scope of the present disclosure.

It will be understood that the embodiments described above are illustrative of some of the applications of the principles of the present subject matter. Numerous modifications may be made by those skilled in the art without departing from the scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. An intermittent urinary catheter product (100), comprising:
a package (102) having opposed front and rear panels (104, 106) that are sealed together to define a sealed interior cavity (103), the package (102) having top and bottom edges (108, 110) and opposed side edges (112,114);
**characterized in that**:
the front panel (104) having an inner surface (105) and the rear panel (106) have an inner surface (107), at least one of the inner surface (105) of the front panel (104) and the inner surface (107) of the rear panel (106) including a reclosable member (116) associated therewith, the reclosable member (116) having a longitudinal axis that is generally parallel with one of the edges;
the front and rear panels (104, 106) of the package (102) being tearable adjacent the reclosable member (116) and in a direction parallel to the reclosable member (116), wherein the tearing of front and rear panels (104, 106) forms an opening (120) in the package (102) adjacent the reclosable member (116) and in communication with the interior cavity (103);
the reclosable member (116) being configured to attach the inner surface (105) of the front panel (104) to the inner surface (107) of the rear panel (106) so as to close the opening (120), the reclosable member (116) including male member (122) comprising a ridge and a female member (124) comprising a groove;
a hydration fluid (140) contained within the interior cavity (103); and
a hydrophilic urinary catheter (123) contained within the interior cavity (103).

2. The intermittent urinary catheter product of Claim 1, wherein the male member (124) is on an opposing inner surface from the female member (124).

3. The intermittent urinary catheter product of Claim 1, wherein the hydrophilic urinary catheter (123) has a distal end including a funnel (132) and a proximal end (134) including a tip (136).

4. The intermittent urinary catheter product of Claim 3, wherein the tip (136) is configured to detachably interconnect with an interior portion of the funnel (134).

5. The intermittent urinary catheter product of Claim 4, wherein the hydrophilic urinary catheter forms a closed loop when the tip (136) and funnel (134) are interconnected.

6. The intermittent urinary catheter product of Claim 1, wherein the hydration fluid (140) is water.

7. The intermittent urinary catheter product of Claim 1, wherein the package (102) has a rectangular shape.

8. The intermittent urinary catheter product of Claim 1, wherein the interior cavity (103) is airtight and watertight.

9. The intermittent urinary catheter product of Claim 1, wherein the package (102) further includes tear elements to facilitate tearing in a desired direction.

10. The intermittent urinary catheter product of Claim 9, wherein the tear elements comprise a tear tape or a tear strip.

11. The intermittent urinary catheter product of Claim 1, wherein the package (102) includes a gripping member (138) comprising a finger hole.

12. The intermittent urinary catheter product of Claim 11, wherein the gripping member (138) is in a sealed portion (138a) of the package (102).

13. The intermittent urinary catheter product of Claim 1, wherein the reclosable member (116) provides a watertight seal for closing the opening (120).

14. The intermittent urinary catheter product of Claim 1, wherein the male member (122) and female member (124) detachably interlock.

15. The intermittent urinary catheter product of Claim 1, wherein the male member (122) and female member (124) have a discontinuous formation.

## Patentansprüche

1. Intermittierendes Harnkatheterprodukt (100), umfassend:
eine Verpackung (102) mit gegenüberliegender Vorder- und Rückwand (104, 106), die miteinander versiegelt sind, um einen versiegelten inneren Hohlraum (103) zu definieren, wobei die Verpackung (102) eine Ober- und eine Unterkante (108, 110) und gegenüberliegende Seitenkanten (112, 114) aufweist;
**dadurch gekennzeichnet, dass**:
die Vorderwand (104) eine Innenfläche (105) und die Rückwand (106) eine Innenfläche (107) haben, mindestens eine von der Innenfläche (105) der Vorderwand (104) und der Innenfläche (107) der Rückwand (106) ein mit ihr verbundenes wiederverschließbares Element (116) aufweist, das wiederverschließbare Element (116) eine Längsachse hat, die allgemein parallel zu einer der Kanten ist;
die Vorder- und die Rückwand (104, 106) der Verpackung (102) benachbart zu dem wiederverschließbaren Element (116) und in einer Richtung parallel zu dem wiederverschließbaren Element (116) aufreißbar sind, wobei das Aufreißen der Vorder- und der Rückwand (104, 106) eine Öffnung (120) in der Verpackung (102) benachbart zu dem wiederverschließbaren Element (116) und in Verbindung mit dem inneren Hohlraum (103) bildet;
wobei das wiederverschließbare Element (116) so konfiguriert ist, dass es die Innenfläche (105) der Vorderwand (104) an der Innenfläche (107) der Rückwand (106) befestigt, um die Öffnung (120) zu verschließen, wobei das wiederverschließbare Element (116) ein Steckelement (122), das einen Steg umfasst, und ein Aufnahmeelement (124), das eine Nut umfasst, aufweist;
ein Hydratationsfluid (140), das im inneren Hohlraum (103) enthalten ist; und
einen hydrophilen Harnkatheter (123), der im inneren Hohlraum (103) enthalten ist.

2. Intermittierendes Harnkatheterprodukt nach Anspruch 1, wobei sich das Steckelement (124) an einer dem Aufnahmeelement (124) gegenüberliegenden Innenfläche befindet.

3. Intermittierendes Harnkatheterprodukt nach Anspruch 1, wobei der hydrophile Harnkatheter (123) ein distales Ende mit einem Trichter (132) und ein proximales Ende (134) mit einer Spitze (136) aufweist.

4. Intermittierendes Harnkatheterprodukt nach Anspruch 3, wobei die Spitze (136) so konfiguriert ist, dass sie abnehmbar mit einem inneren Abschnitt des Trichters (134) verbunden ist.

5. Intermittierendes Harnkatheterprodukt nach Anspruch 4, wobei der hydrophile Harnkatheter einen geschlossenen Kreis bildet, wenn die Spitze (136) und der Trichter (134) miteinander verbunden sind.

6. Intermittierendes Harnkatheterprodukt nach Anspruch 1, wobei das Hydratationsfluid (140) Wasser ist.

7. Intermittierendes Harnkatheterprodukt nach Anspruch 1, wobei die Verpackung (102) eine rechteckige Form hat.

8. Intermittierendes Harnkatheterprodukt nach Anspruch 1, wobei der innere Hohlraum (103) luft- und wasserdicht ist.

9. Intermittierendes Harnkatheterprodukt nach Anspruch 1, wobei die Verpackung (102) ferner Aufreißelemente aufweist, die das Aufreißen in einer gewünschten Richtung erleichtern.

10. Intermittierendes Harnkatheterprodukt nach Anspruch 9, wobei die Aufreißelemente ein Aufreißband oder einen Aufreißstreifen umfassen.

11. Intermittierendes Harnkatheterprodukt nach Anspruch 1, wobei die Verpackung (102) ein Griffelement (138) aufweist, das ein Fingerloch umfasst.

12. Intermittierendes Harnkatheterprodukt nach Anspruch 11, wobei sich das Greifelement (138) in einem versiegelten Abschnitt (138a) der Verpackung (102) befindet.

13. Intermittierendes Harnkatheterprodukt nach Anspruch 1, wobei das wiederverschließbare Element (116) eine wasserdichte Abdichtung zum Verschließen der Öffnung (120) bereitstellt.

14. Intermittierendes Harnkatheterprodukt nach Anspruch 1, wobei das Steckelement (122) und das Aufnahmeelement (124) lösbar ineinandergreifen.

15. Intermittierendes Harnkatheterprodukt nach Anspruch 1, wobei das Steckelement (122) und das Aufnahmeelement (124) eine diskontinuierliche Formation aufweisen.

## Revendications

1. Produit de cathéter urinaire intermittent (100), comprenant :
un emballage (102) présentant des panneaux avant et arrière (104, 106) opposés scellés l'un à l'autre pour définir une cavité intérieure (103) hermétique, l'emballage (102) présentant des bords supérieur et inférieur (108, 110) et des bords latéraux (112, 114) opposés ;
**caractérisé en ce que** :
le panneau avant (104) présentant une surface interne (105) et le panneau arrière (106) présentant une surface interne (107), au moins l'une de la surface interne (105) du panneau avant (104) et de la surface interne (107) du panneau arrière (106) comportant un élément refermable (116) associé à ceux-ci, l'élément refermable (116) présentant un axe longitudinal qui est généralement parallèle à l'un des bords ;
les panneaux avant et arrière (104, 106) de l'emballage (102) pouvant être déchirés à proximité de l'élément refermable (116) et dans une direction parallèle à l'élément refermable (116), dans lequel la déchirure des panneaux avant et arrière (104, 106) forme une ouverture (120) dans l'emballage (102) à proximité de l'élément refermable (116) et en communication avec la cavité intérieure (103) ;
l'élément refermable (116) étant configuré pour fixer la surface interne (105) du panneau avant (104) à la surface interne (107) du panneau arrière (106) de manière à fermer l'ouverture (120), l'élément refermable (116) comportant un élément mâle (122) comprenant une nervure et un élément femelle (124) comprenant une rainure ;
un fluide d'hydratation (140) contenu à l'intérieur de la cavité intérieure (103) ; et
un cathéter urinaire hydrophile (123) contenu à l'intérieur de la cavité intérieure (103).

2. Produit de cathéter urinaire intermittent selon la revendication 1, dans lequel l'élément mâle (124) se situe sur une surface interne opposée à l'élément femelle (124).

3. Produit de cathéter urinaire intermittent selon la revendication 1, dans lequel le cathéter urinaire hydrophile (123) présente une extrémité distale comportant un entonnoir (132) et une extrémité proximale (134) comportant une pointe (136).

4. Produit de cathéter urinaire intermittent selon la revendication 3, dans lequel la pointe (136) est configurée pour s'interconnecter de manière amovible avec une partie intérieure de l'entonnoir (134).

5. Produit de cathéter urinaire intermittent selon la revendication 4, dans lequel le cathéter urinaire hydrophile forme une boucle fermée lorsque la pointe (136) et l'entonnoir (134) sont interconnectés.

6. Produit de cathéter urinaire intermittent selon la revendication 1, dans lequel le fluide d'hydratation (140) est de l'eau.

7. Produit de cathéter urinaire intermittent selon la revendication 1, dans lequel l'emballage (102) présente une forme rectangulaire.

8. Produit de cathéter urinaire intermittent selon la revendication 1, dans lequel la cavité intérieure (103) est étanche à l'air et à l'eau.

9. Produit de cathéter urinaire intermittent selon la revendication 1, dans lequel l'emballage (102) comporte également des éléments de déchirure pour faciliter la déchirure dans une direction souhaitée.

10. Produit de cathéter urinaire intermittent selon la revendication 9, dans lequel les éléments de déchirure comprennent un ruban de déchirure ou une bande de déchirure.

11. Produit de cathéter urinaire intermittent selon la revendication 1, dans lequel l'emballage (102) comporte un élément de préhension (138) comprenant un trou pour le doigt.

12. Produit de cathéter urinaire intermittent selon la revendication 11, dans lequel l'élément de préhension (138) se situe dans une partie hermétique (138a) de l'emballage (102).

13. Produit de cathéter urinaire intermittent selon la revendication 1, dans lequel l'élément refermable (116) procure un joint étanche à l'eau destiné à fermer l'ouverture (120).

14. Produit de cathéter urinaire intermittent selon la revendication 1, dans lequel l'élément mâle (122) et l'élément femelle (124) s'emboîtent de manière amovible.

15. Produit de cathéter urinaire intermittent selon la revendication 1, dans lequel l'élément mâle (122) et l'élément femelle (124) sont de forme discontinue.
